(19) Europäisches Patentamt
European Patent Office
Office européen des brevets

(11) **EP 3 004 363 B1**

(12) **EUROPEAN PATENT SPECIFICATION**

(45) Date of publication and mention
of the grant of the patent:
**01.08.2018 Bulletin 2018/31**

(51) Int Cl.:
**C12P 7/46** (2006.01)   **C12P 5/02** (2006.01)

(21) Application number: **14727795.8**

(22) Date of filing: **26.05.2014**

(86) International application number:
**PCT/EP2014/060807**

(87) International publication number:
**WO 2014/188000 (27.11.2014 Gazette 2014/48)**

(54) **METHOD FOR UPGRADING OF A FUEL GAS DURING SUCCINIC ACID PRODUCTION**

VERFAHREN ZUR VEREDELUNG EINES BRENNGASES WÄHREND DER BERNSTEINSÄUREHERSTELLUNG

PROCÉDÉ DE VALORISATION D'UN GAZ COMBUSTIBLE AU COURS DE LA PRODUCTION D'ACIDE SUCCINIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2013 EP 13169127**

(43) Date of publication of application:
**13.04.2016 Bulletin 2016/15**

(73) Proprietor: **Technical University of Denmark
2800 Kgs. Lyngby (DK)**

(72) Inventors:
• **ANGELIDAKI, Irini**
  **DK-2000 Frederiksberg (DK)**
• **BRAGI GUNNARSSON, Ingólfur**
  **DK-2450 Copenhagen (DK)**
• **ALVARADO-MORALES, Merlin**
  **DK-2800 Kgs. Lyngby (DK)**

(74) Representative: **Høiberg P/S
Adelgade 12
1304 Copenhagen K (DK)**

(56) References cited:
• **NGHIEM ET AL: "Integration of succinic acid and ethanol production with potential application in a corn or barley refinery", APPLIED BIOCHEMISTRY AND BIOTECHNOLOGY, vol. 162, 2010, pages 1915-1928, XP002711227,**

• **LU ET AL: "Effects of flue gas components on succinate production in dual-phase Escherichia coli fermentations", 30th Symposium on Biotechnology for Fuels and Chemicals / New Orleans, LA, 2008, page 1, XP002711283, Retrieved from the Internet: URL:http://sim.confex.com/sim/30th/techprogram/P5512.HTM [retrieved on 2013-08-14]**
• **"BASF und CSM gründen 50-50 Joint Venture für biobasierte Bernsteinsäure", Press release, 5 October 2012 (2012-10-05), pages 1-3, XP002711284, Retrieved from the Internet: URL:http://co2-chemistry.eu/news/basf-und-csm-grunden-50-50-joint-venture-fur-biobasierte-bernsteinsaure/ [retrieved on 2013-08-13]**
• **LUO ET AL: "Biorefining of lignocellulosic feedstock - technical, economic and environmental considerations", BIORESOURCE TECHNOLOGY, vol. 101, 2010, pages 5023-5032, XP026986242,**
• **LUO ET AL: "Integrated biogas upgrading and hydrogen utilization in an anaerobic reactor containing enriched hydrogenotrophic methanogenic culture", BIOTECHNOLOGY AND BIOENGINEERING, vol. xxx, 21 May 2012 (2012-05-21), pages 1-8, XP002711228,**
• **CHENG ET AL: "Biotechnological production of succinic acid: current state and perspectives", BIOFUELS, BIOPRODUCTS & BIOREFINING, vol. 6, 29 February 2012 (2012-02-29), pages 302-308, XP055091914,**

- BAUER ET AL: "Biogas upgrading - technology overview, comparison and perspectives for the future", BIOFUELS, BIOPRODUCTS & BIOREFINING, 2013, pages 1-13, XP002711234,
- BRAGI GUNNARSSON ET AL: "Production of succinic acid by fermentation", Afternoon Seminar on Biorefinery in the Öresund Region; June 2013, 2013, pages 1-4, XP002726255, Retrieved from the Internet: URL:http://www.env.dtu.dk/english/About/Calender/2013/06/2013-June-Seminar-Biorefinery-Resources-Engineering-Oeresund-Region [retrieved on 2014-06-26]
- N.N.: "Flue gases and fuel gases", ZEVENHOVEN & KILPINEN Chapter 2, 2001, pages 1-12, Retrieved from the Internet: URL:http://users.abo.fi/rzevenho/gases.PDF

**Description**

**Technical Field**

The present invention relates to the field of upgrading of a CO2-containing fuel gas such as biogas by converting the $CO_2$ content of the fuel gas to succinic acid by use of succinic-acid producing bacteria.

**Background**

The production and utilization of biogas by anaerobic digestion of organic wastes is an emerging alternative energy technology. Biogas is envisioned as a key element in emerging renewable energy strategies in Europe, motivated by the European Union target of achieving 20% renewable energy by 2020. The Danish government also proposed a target of using 50% of the manure produced in Denmark for renewable energy production by 2020, and it would essentially be met through a strong biogas expansion.

Biogas mainly contains methane or $CH_4$ (50-75%) and carbon dioxide or $CO_2$ (25-50%). For biogas to be used as fuel it must be upgraded. The upgrading process removes unwanted gases from the biogas, mainly $CO_2$, but also other gas impurities. Upgrading of biogas to $CH_4$ content higher than 90% can not only increase the heating value, but also reduce corrosion caused by acid gas and therefore extend the biogas utilization as a renewable energy source.

Conventional methods of fuel gas upgrading include water washing, pressure swing adsorption, polyglycol adsorption and chemical treatment, which aim to remove $CO_2$ from the biogas. The costs of the above methods are relatively high since they need either high pressure or addition of chemicals. Besides, when removing $CO_2$ from biogas, small amounts of $CH_4$ are also removed, which will possibly increase the greenhouse gas emission.

Another approach is to apply anaerobic microorganisms to convert $CO_2$ to $CH_4$ for biogas upgrading, by simultaneous $H_2$ consumption (equation 1):

$$4\,H_2 + CO_2 \rightarrow CH_4 + 2H_2O \qquad\qquad \Delta G^0 = -130.7\text{KJ/mol} \qquad\qquad (1)$$

This process is facilitated by methanogenic organisms preferably cultured at a pH of between 7-8. The process includes adding $H_2$ containing gas to a biogas reactor. However, as the process progresses, the $CO_2$ of the culture medium is consumed resulting in an increased pH of the culture medium; when pH exceeds 8.3 the process is inhibited as the culturing conditions for the methanogenic organism has become sub-optimal. This process is disclosed e.g. in WO 2013/060331. Nghiem et al, Applied Biochemistry and Biotechnology (2010) 162:1915-1928, disclose production of succinic acid from glucose with and without carbon dioxide. Luo et al, Biotechnology and Bioengineering (2012) 109:2729-2736, disclose biogas upgrading in a biogas reactor containing hydrogenotrophic methanogens, such as Methanobacteriales, to convert $CO_2$ to $CH_4$. Lu et al, 30th Symposium on Biotechnology for Fuels and Chemicals, 2008, New Orleans, discuss effects of flue gas from power plants on succinate production. Further, Gunnarsson et al, Afternoon Seminar on Biorefinery in the Oresund region, June 2013, presented parts of the present method. Alternative methods for upgrading $CO_2$-containing fuel gases are required.

**Summary**

Biogas produced through anaerobic digestion of organic matter is a gas mixture containing 50-75% methane ($CH_4$) and 25-50% carbon dioxide ($CO_2$).

Biogas is currently one of the fastest growing sources of energy. However as biogas is produced from biomass and contains a high proportion of $CO_2$ which has a low value as energy source. Thus it is desirable to decrease the content of $CO_2$ in biogas by a process often referred to as upgrading of biogas. The most common upgrading methods remove the impurities mechanically or chemically.

Other fuel gases, such as syngas and coal gas, also comprise $CO_2$.

Until recently, the common industrial scale production method of succinic acid was petroleum derived. Emerging 'green' technologies to produce bio-based succinic acid have a lower operating cost and a more environmentally friendly foot print. These exploit biocatalyst driven fermentation of a carbon source.

The present invention provides a biological process of fermenting a carbon source to succinic acid, by exploiting the simultaneous consumption or fixation of $CO_2$ of a $CO_2$-containing fuel gas by anaerobic succinic acid-producing microorganisms. This process thus provides for simultaneous fuel gas upgrading and succinic acid production, both of which are commercially relevant products of considerable value.

It is thus an embodiment of the present invention to provide a method of manufacturing an upgraded fuel gas, and simultaneously producing succinic acid, said method comprising the steps of:

a. providing a bioreactor,
b. providing anaerobic succinic acid-producing microorganisms,
c. providing a carbon based substrate for said anaerobic succinic acid-producing microorganisms,
d. adding a $CO_2$-containing fuel gas to the bioreactor,
e. collecting the upgraded fuel gas thus produced, wherein said upgraded fuel gas has a lower $CO_2$ content than the $CO_2$-containing fuel gas added, and
f. collecting the effluent containing succinic acid.

In one embodiment said $CO_2$-containing fuel gas is selected from the group consisting of biogas, syngas and coal gas. In one embodiment said method comprising the steps of:

a. providing a bioreactor,
b. providing anaerobic succinic acid-producing organisms,
c. providing a carbon based substrate for said anaerobic succinic acid-producing organisms,
d. adding biogas to the bioreactor,
e. collecting the upgraded biogas thus produced, and
f. optionally collecting the effluent containing succinic acid.

In one embodiment said anaerobic succinic acid-producing microorganism is an anaerobic succinic acid-producing bacteria; in one embodiment said bacteria is *Actinobacillus succinogenes.*

In one embodiment said carbon based substrate is a carbohydrate substrate comprising one or more of glucose, xylose, arabinose, galactose, maltose, fructose, sucrose, cellobiose, lactose, mannitol, arabitol, sorbitol, mannose, ribose, glycerol, pectin, beta-glucoside, gluconate, idonate, ascorbate, glucarate, galactarate and/or 5-keto-glucanate.

In one embodiment the method further comprises the step of recovering and/or purifying succinic acid from the effluent containing succinic acid.

The method according to the present invention offers several advantages, as it could prove to be more energy efficient than current technologies for upgrading of $CO_2$-containing fuel gas and potentially result in lower investment and operational costs than conventional upgrading technologies.

Furthermore, the succinic acid by-product of the present method of fuel gas upgrading is a marketable product in its own right, with a growing market potential. Compared to the conventional petroleum-based succinic acid production process the method has a potential to improve the environmental footprint and at the same time promote bio-based economy.

The upgraded biogas is preferably upgraded in order to be utilised in existing gas grid infrastructure.

**Description of Drawings**

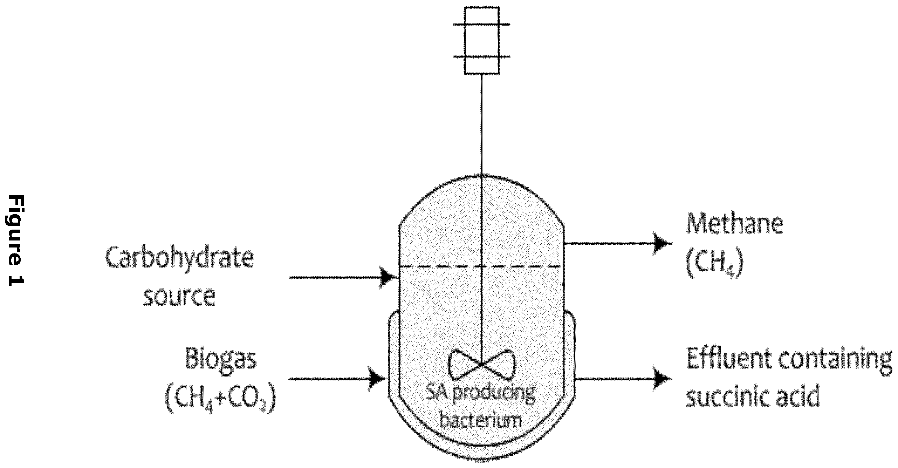

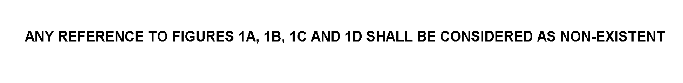

Figure 1: Schematic biogas upgrading reactor.

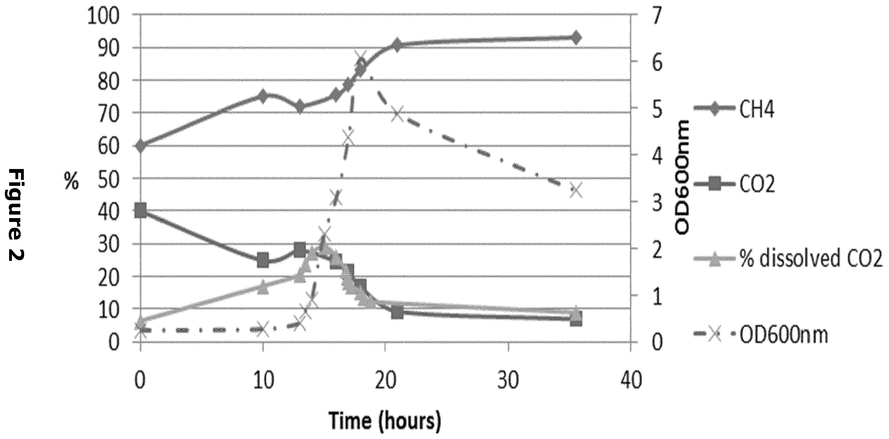

Figure 2: Biogas upgrading. The bacterial growth (OD660nm) was monitored for 36 hours. During bacterial growth biogas composition in gas phase (w. GC) and solubilized $CO_2$ (w. in-situ online $CO_2$ sensor) was measured. As observed in this figure the biogas in this experiment is upgraded from 60/40 composition to 90/10 composition.

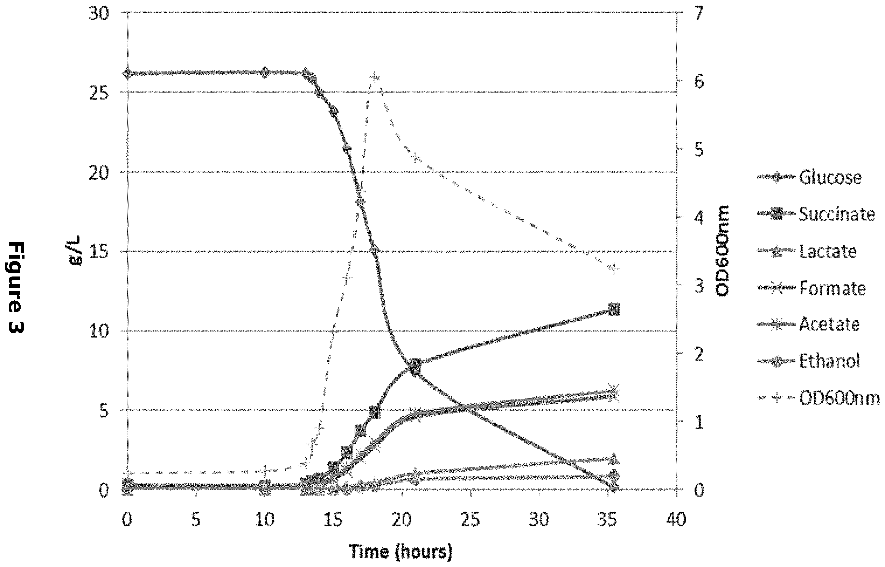

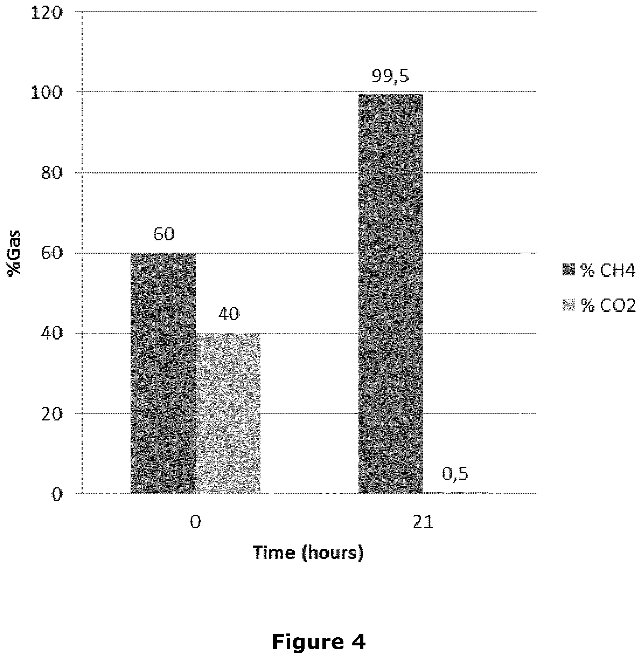

Figure 3: A. succinogenes growth and metabolites. Bacterial growth (OD660) identical to figure 4. The consumption of glucose and the production of succinate, lactate, formate (methanoate), acetate and ethanol were measured using HPLC. The presence of lactate indicates presence of small amounts of $O_2$. After observing the presence of lactate, the tubing in our system was changed to air tight tubes. After that, no lactate has been observed. With recent optimisations we now have the system running where no lactate is produced.

Figure 4: Biogas upgrading results. Comparison of biogas composition before and after upgrading (21 hours). Only trace amounts of $CO_2$ were observed in the gas after upgrading. As shown in the plot all the biogas is upgraded after 21 hours when the total gas volume in the system is 6.7L and glucose present in excess. Highest succinic acid yield so far reached using biogas as a source of $CO_2$ is 0.61 g SA/g glucose. However highest yield using MgCO3 as a $CO_2$ source was 0.85 g SA/g glucose.

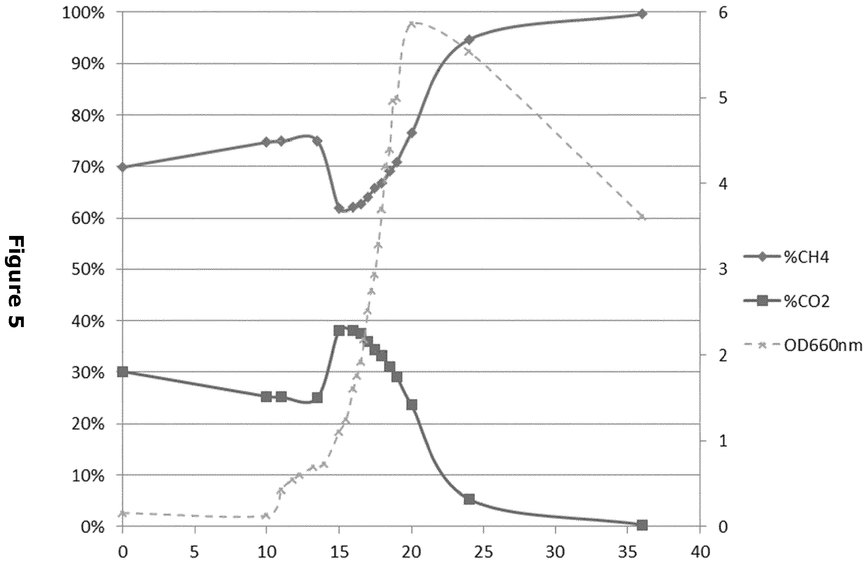

Figure 5: Biogas upgrading of 6.7L of biogas (GC). Using succinic acid fermentation the biogas was upgraded to 99.6% $CH_4$/0.4% $CO_2$.

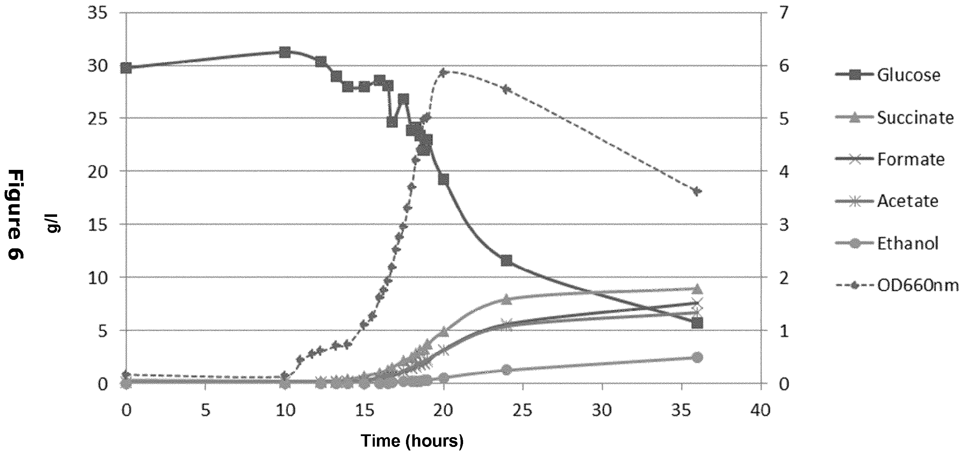

Figure 6: Succinic acid fermentation during biogas upgrading (HPLC). All glucose was not consumed due to depletion of $CO_2$. The ration between succinate and other metabolites decreases as $CO_2$ levels decrease. The succinic acid production in this experiment was as expected, lower succinic acid yields are reached when $CO_2$ levels are low. The yield observed was 0.37 g SA/g glucose. The succinic acid production is highly dependent on the availability of $CO_2$. However, during fermentation the biogas was upgraded to 99.6% $CH_4$ and only 0.4% $CO_2$ remaining.

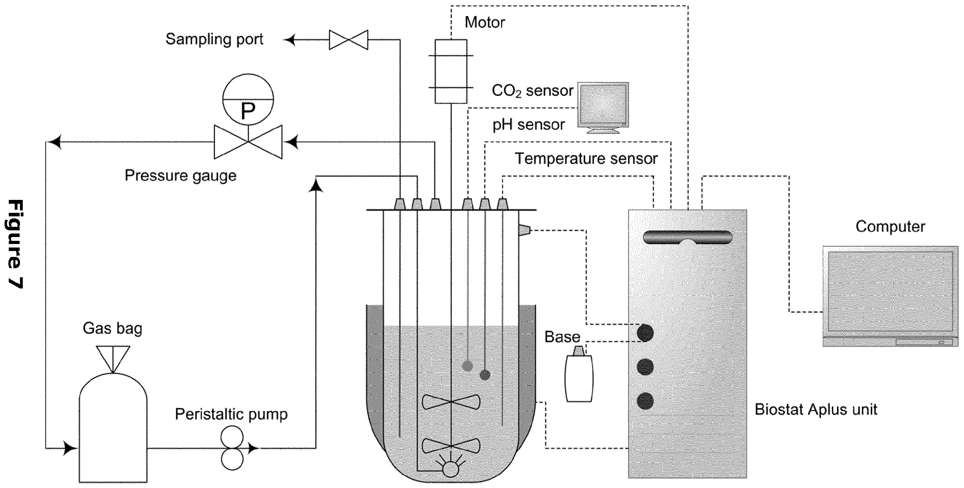

Figure 7: 3-L bioreactors (Sartorius BIOSTAT Aplus, Germany) with an initial working volume of 1.5 L were used. The bioreactors were equipped with EASYFERM PLUS K8 200 pH probes (Hamilton Bonaduz AG, Switzerland), temperature and CO2 (Mettler Toledo, Switzerland). Flowrate of gas mixtures was controlled through a peristaltic pump (Watson-Marlow, United Kingdom). For monitoring and controlling pressure inside bioreactors a pressure gauge (Cewai, Italy) was mounted with a relief valve (Hy-Lok, USA) set to the experimental pressure. Different size gas bags (7.5 and 12.5 L) were used to supply gas to the system. The system consists of a gas recirculation which allows observing changes in biogas composition during fermentation.

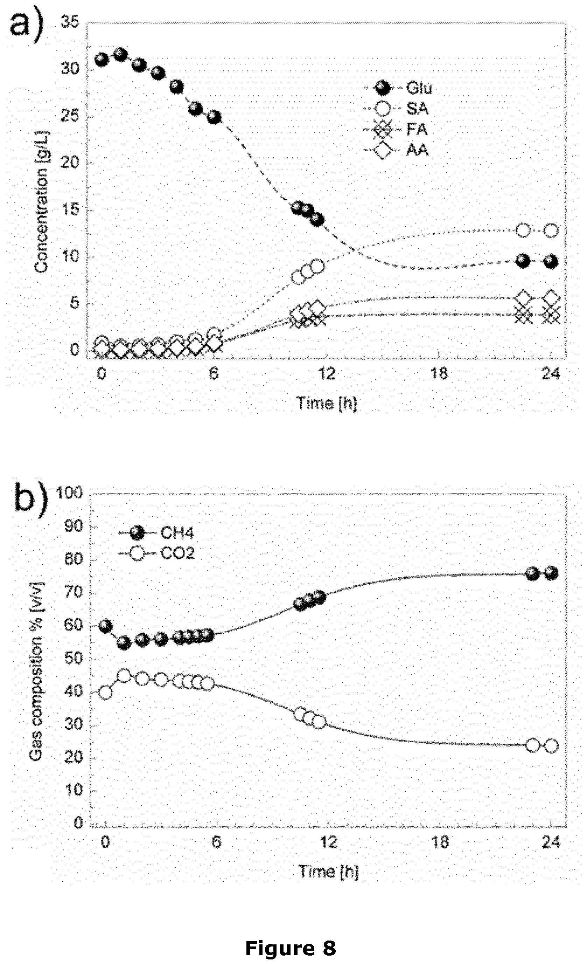

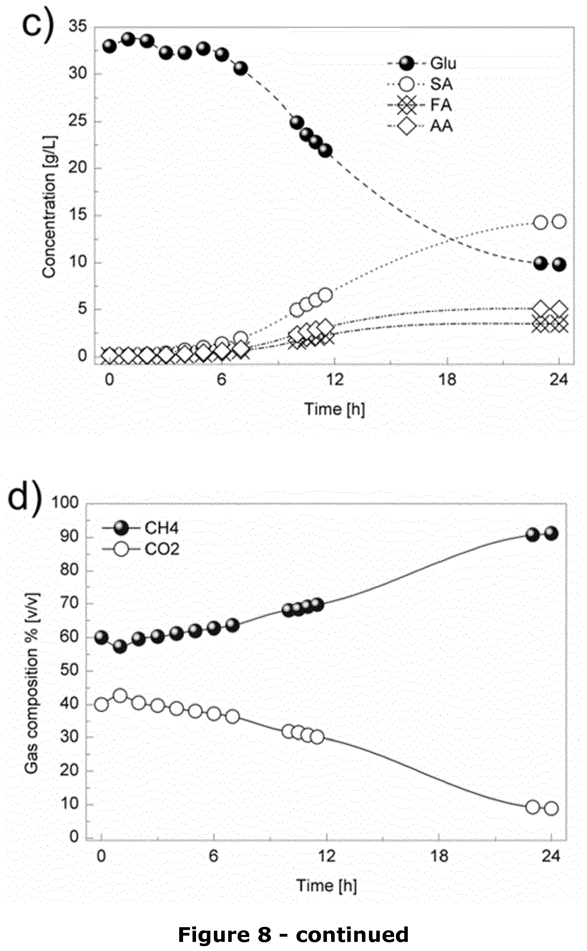

Figure 8: Fermentation was carried out at two different conditions. Firstly, at atmospheric pressure where Figure 1a) shows fermentation products and Figure 1b) shows the biogas composition. Secondly, at 1.6 bar pressure Figure 1c) shows fermentation products and Figure 1d) shows the biogas composition. The biogas volume used in this experiment was 12.5 liters. Succinic acid concentration was increased by 11% (from 12.85 g/L to 14.39 g/L) when fermentation at atmospheric pressure was compared to fermentation performed at 1.6 bar. The succinic acid yield increased by 4% (from 0.597 g/g to 0.621 g/g) when the two experimental conditions were compared. When comparing the composition of biogas at the end of fermentation, due to higher succinic acid production, less by-product formation and higher solubility of CO2 the methane content was increased from being 76% (fermentation at atmospheric pressure) to 91% (fermentation at 1.6 bar pressure). *Glu = Glucose, SA = Succinic acid, FA = Formic acid, AA = Acetic acid

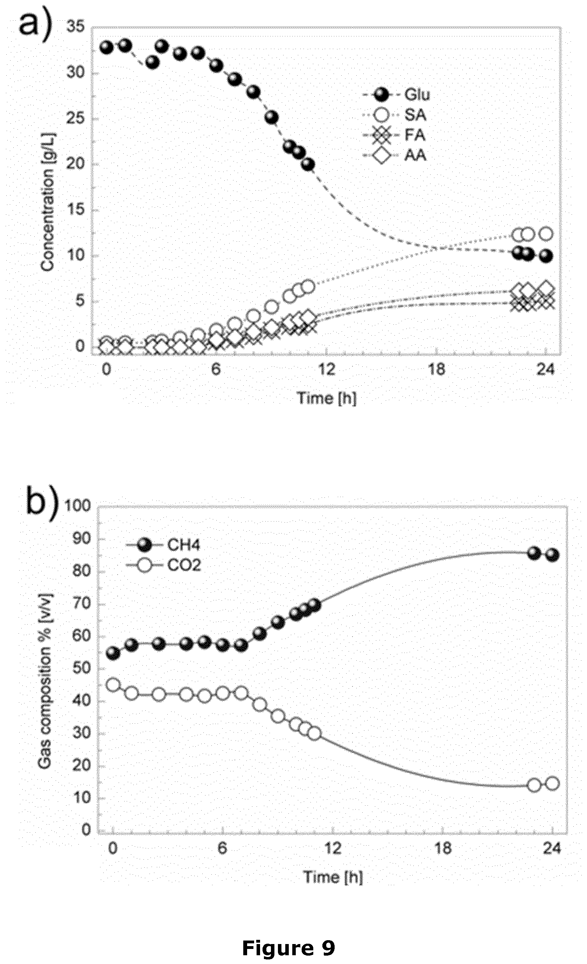

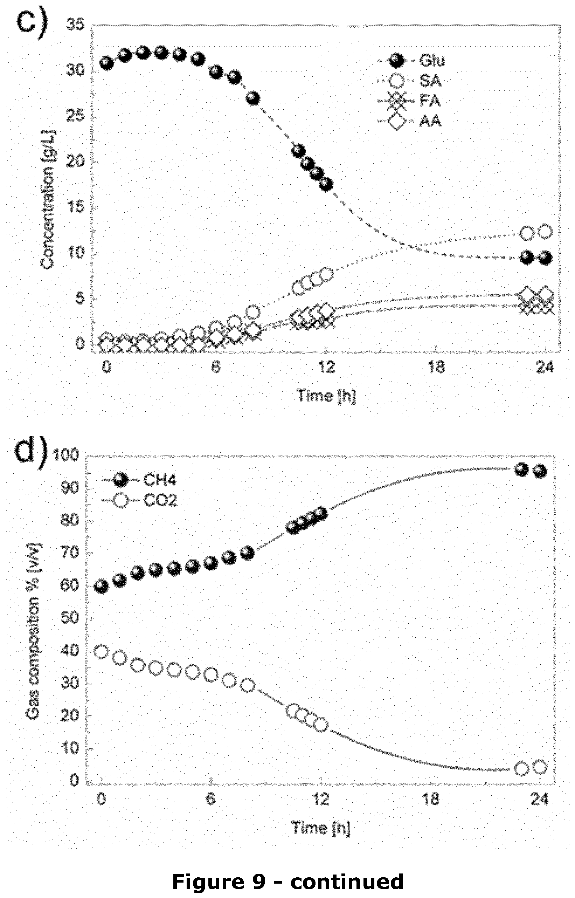

Figure 9: Fermentation was carried out at two different conditions. Firstly, at atmospheric pressure where Figure 1a) shows fermentation products and Figure 1b) shows the biogas composition. Secondly, at 1.6 bar pressure Figure 1c) shows fermentation products and Figure 1d) shows the biogas composition. The biogas volume used in this experiment was 7.5 liters. Succinic acid concentration did not increase when fermentation at atmospheric pressure was compared to fermentation performed at 1.6 bar. However, the succinic acid yield increased by 7% (from 0.545 g/g to 0.585 g/g) when the two experimental conditions were compared. When comparing the composition of biogas at the end of fermentation, due to less gas volume (volume decreased from 12.5L to 7.5L as compared to figure 8), less by-product formation and higher solubility of CO2 the methane content was increased from being 85% (fermentation at atmospheric pressure) to 95% (fermentation at 1.6 bar pressure). *Glu = Glucose, SA = Succinic acid, FA = Formic acid, AA = Acetic acid

**Detailed description**

The present invention is as defined in the claims.

Fuel gas is any one of a number of fuels that under ordinary conditions are gaseous. Many fuel gases are composed of hydrocarbons (such as methane or propane), hydrogen, carbon monoxide, or mixtures thereof. Such gases are sources of potential heat energy or light energy that can be readily transmitted and distributed through pipes from the point of origin directly to the place of consumption.

Fuel gas is contrasted with liquid fuels and from solid fuels, though some fuel gases are liquefied for storage or transport. The most common type of fuel gas in current use is natural gas composed primarily of methane.

Fuel gas may be classified into manufactured gases (produced via an artificial process, usually gasification at gasworks) and those naturally occurring (including natural gas and petroleum gases). Manufactured gases include coal gas, water gas, producer gas, syngas, wood gas, hydrogen and biogas.

Syngas, or synthesis gas, is a fuel gas mixture consisting primarily of hydrogen ($H_2$), carbon monoxide (CO), and very often some carbon dioxide ($CO_2$). The hydrogen must be separated from the $CO_2$ to be able to use it. This is primarily done by pressure swing adsorption (PSA), amine scrubbing, and membrane reactors.

Coal gas (also town gas and illumination gas) is a flammable gaseous fuel made by the destructive distillation of coal and contains a variety of calorific gases including hydrogen, carbon monoxide, methane and volatile hydrocarbons together with small quantities of non-calorific gases such as carbon dioxide and nitrogen.

Biogas

Biogas is a sustainable source of energy and is a type of biofuel. Biogas is produced by the anaerobic digestion of organic matter (biomass) such as manure, sewage, municipal waste, green waste, plant material, animal material, kitchen waste and crops. Biogas comprises primarily methane ($CH_4$) and carbon dioxide ($CO_2$) and may have small amounts of hydrogen sulphide ($H_2S$), moisture and siloxanes.

The gases methane, hydrogen, and carbon monoxide (CO) can be combusted. Energy release through said combustion allows biogas to be used as a fuel. Biogas can be used as a fuel in any country for any heating purpose, such as cooking. It can also be used in anaerobic digesters where it is typically used in a gas engine to convert the energy in the gas into electricity and heat. Biogas can be compressed, much like natural gas, and used to power motor vehicles. In the UK, for example, biogas is estimated to have the potential to replace around 17% of vehicle fuel. Biogas is a renewable fuel, so it qualifies for renewable energy subsidies in some parts of the world. Biogas can also be cleaned and upgraded to natural gas standards (biomethane).

The composition of biogas varies depending upon the biomass and origin used for the anaerobic digestion process. However the composition of raw biogas produced by traditional methods typically ranges within the values summarized in table 1 below:

**Table 1**

| Compound | Formula | % |
|---|---|---|
| Methane | $CH_4$ | 50-75 |
| Carbon dioxide | $CO_2$ | 25-50 |
| Nitrogen | $N_2$ | 0-10 |
| Hydrogen | $H_2$ | 0-1 |
| Hydrogen sulfide | $H_2S$ | 0-3 |
| Oxygen | $O_2$ | 0-0 |

The composition of biogas varies depending upon the origin of the anaerobic digestion process. Landfill gas typically has methane concentrations around 50%. Advanced waste treatment technologies can produce biogas with 55-75% $CH_4$, which for reactors with free liquids can be increased to 80-90% methane using in-situ gas purification techniques.

When biogas is used, many advantages arise. In North America, utilization of biogas would generate enough electricity to meet up to three per cent of the continent's electricity expenditure. In addition, biogas could potentially help reduce global climate change. Normally, manure that is left to decompose releases two main gases that cause global climate change: nitrous dioxide and methane. Nitrous dioxide ($N_2O$) warms the atmosphere 310 times more than carbon dioxide and methane 21 times more than carbon dioxide. By converting cow manure into methane biogas via anaerobic digestion, the millions of cows in the United States would be able to produce one hundred billion kilowatt hours of electricity, enough to power millions of homes across the United States. In fact, one cow can produce enough manure in one day to generate three kilowatt hours of electricity; only 2.4 kilowatt hours of electricity are needed to power a single one hundred watt light bulb for one day. Furthermore, by converting cow manure into methane biogas instead of letting it decompose, it would be possible to reduce global warming gases by ninety-nine million metric tons or four per cent.

*Biogas utilisation*

Biogas can be utilized for electricity production on sewage works in a CHP gas engine, where the waste heat from the engine is conveniently used for heating the digester; cooking; space heating; water heating; and process heating. If compressed, it can replace compressed natural gas for use in vehicles, where it can fuel an internal combustion engine or fuel cells and is a much more effective displacer of carbon dioxide than the normal use in on-site CHP plants.

Methane within biogas can be concentrated via a biogas upgrader to the same standards as fossil natural gas, which itself has had to go through a cleaning process, and becomes biomethane. If the local gas network allows for this, the producer of the biogas may utilize the local gas distribution networks. Gas must be very clean to reach pipeline quality, and must be of the correct composition for the local distribution network to accept. Carbon dioxide, water, hydrogen sulfide, and particulates must be removed if present.

The requirements to the purity and composition of the biogas for distribution in the natural gas grid infrastructure vary in different countries. For instance, the Danish national requirements to biogas include e.g. a wobbe index in the range of 50.8 to 55.8 MJ/Nm$^3$ and a $CO_2$ content less than 2.5%. For example, a biogas with a $CH_4$ content of 97.3% has an acceptable wobbe index and may be used directly in the Danish natural gas grid infrastructure.

In one embodiment as disclosed herein, the fuel gas upgraded by the present method is added propane to adjust the burn value (wobbe index) of the gas. Conventional methods for upgrading fuel gases are listed in table 2 below:

**Table 2**

| Technique | Investment cost ($\epsilon$) | Running cost ($\epsilon$) | cost price upgraded biogas ($\epsilon/Nm^3$) | Maximum achievable yield (%) | Maximum achievable purity (%) |
|---|---|---|---|---|---|
| Chemical absorption | 353,000 | 134,500 | 0.17 | 90 | 98 |
| High pressure water scrubbing | 265,000 | 110,000 | 0.13 | 94 | 98 |
| Pressure swing absorbtion | 680,000 | 187,250 | 0.25 | 91 | 98 |
| Cryogenic separation | 908,500 | 397,500 | 0.44 | 98 | 91 |
| Membrane separation | 233,000 | 81,750 | 0.12 | 78 | 89.5 |

The costs of the above methods are relatively high since they need either high pressure or addition of chemicals. Besides, when removing $CO_2$ from biogas, small amounts of $CH_4$ are also removed.

Succinic acid

Succinic acid (butanedioic acid; historically known as spirit of amber) is a diprotic, dicarboxylic acid with chemical formula $C_4H_6O_4$ and structural formula HOOC-$(CH_2)_2$-COOH. It is a white, odorless solid. Succinate plays a role in the citric acid cycle, an energy-yielding process. The name derives from Latin *succinum,* meaning amber, from which the acid was originally obtained.

In the past it was chiefly used externally for rheumatic aches and pains, and internally in inveterate gleets. Succinic acid is a precursor to some specialized polyesters. It is also a component of some alkyd resins. Succinic acid is used in the food and beverage industry, primarily as an acidity regulator. In nutraceutical form as a food additive and dietary supplement, is safe and approved by the U.S. Food and Drug Administration. As an excipient in pharmaceutical products it is used to control acidity and, more rarely, in effervescent tablets.

Salts formed by neutralizing succinic acid are called succinates. One example is sodium succinate, a white, water-soluble salt. Esters of succinic acid are also called succinates. Succinate and succinic acid may be used interchangeably herein.

Succinic acid can be produced by several methods. Common industrial routes include hydrogenation of maleic anhydride and hydration of succinic anhydride; hydration of maleic anhydride and hydrogenation of maleic acid; oxidation of 1,4-butanediol, and carbonylation of ethylene glycol. The former processes are denoted the petrochemical based route.

Emerging techniques provide a more environmentally friendly process for producing succinic acid made from renewable, sustainable sources. Bio-based succinic acid (biosuccinic acid) is made from fermentation of a carbon source such as a carbohydrate (e.g. glucose), followed by a recovery and purification step. The process is anaerobic in nature and consumes $CO_2$ rather than releasing it. The process is mediated by a biocatalyst microorganism.

Global production is estimated at 30,000 tons a year, with an annual growth rate of 10%. With the introduction of biosuccinic acid the market is expected to expand.

*A. succinogenes* and *M. succiniciproducens* are bacteria that naturally convert sugars and $CO_2$ into high concentrations of succinic acid as part of a mixed-acid fermentation. Efforts are ongoing to maximize carbon flux to succinate to achieve an industrially applicable process (McKinlay et al., 2010).

*Succinic acid fermentation stoichiometry*

Theoretically 1 mole $CO_2$ is consumed per mole succinic acid produced as illustrated in the chemical formula of glucose fermentation into succinic acid. The theoretical chemical yield for succinic acid is (equation 2):

$$C_6H_{12}O_6 + CO_2 \rightarrow HOOC - CH_2 - CH_2 - COOH + CH_3COOH + HCOOH$$

~~Methods according to the present disclosure~~

As mentioned herein above raw biogas produced from digestion in traditional biogas reactors is roughly 60% methane and 40% $CO_2$. The high $CO_2$ content makes the biogas less attractive for direct use as energy source. The solution is the use of a biogas upgrading or purification process whereby contaminants in the raw biogas stream are absorbed or scrubbed, leaving up to 98% methane per unit volume of gas. Traditionally there have been four main methods of biogas upgrading including water washing, pressure swing absorption, selexol absorption, and amine gas treating. The most prevalent method is water washing where high pressure gas flows into a column where the carbon dioxide and other trace elements are scrubbed by cascading water running counter-flow to the gas. Present day arrangements, however, are expensive and impractical as they require a further step before the biogas can be utilised. The present inventors have demonstrated that it is possible to obtain large quantities of high-grade (high content $CH_4$) biogas by making use of a relatively simple biological process for consuming $CO_2$ in the biogas in the process of making succinic acid. The upgraded biogas can be utilised in an existing natural gas grid infrastructure and/or directly as a fuel source.

While it is known that certain microorganisms can produce succinic acid, it has not previously been suggested that anaerobic succinic acid producing microorganisms could be employed for upgrading of fuel gas by consumption or fixation of the $CO_2$ content of the fuel gas and the simultaneous production of succinic acid. Thus, two commercially relevant products (upgraded fuel gas and succinic acid) are obtained in one reaction.

It is an aspect of the invention to provide

- a method of upgrading a $CO_2$-containing fuel gas, such as biogas,
- a method of producing succinic acid, and
- a method of simultaneously upgrading a $CO_2$-containing fuel gas, such as biogas, and producing succinic acid,

said methods, as defined in the claims, comprising providing anaerobic succinic acid-producing organisms, providing a carbohydrate substrate for said anaerobic succinic acid-producing organisms, and providing a $CO_2$-containing fuel gas such as biogas.

Preferably, the reactions utilised in said methods will take place in a bioreactor suitable for containing gas and a fermentation broth and for providing an anaerobic milieu.

It is thus an embodiment to provide a method of manufacturing an upgraded fuel gas, said method comprising the steps of:

a. providing a bioreactor,
b. providing anaerobic succinic acid-producing organisms,
c. providing a carbon based substrate for said anaerobic succinic acid-producing organisms,
d. adding a $CO_2$-containing fuel gas to the bioreactor,
e. collecting the upgraded fuel gas thus produced, wherein said upgraded fuel gas has a lower $CO_2$-content than the $CO_2$-containing fuel gas added, and
f. optionally collecting the effluent containing succinic acid.

The term 'upgraded fuel gas' as used herein and according to the present disclosure means that the incombustible component or non-calorific component of the fuel gas is reduced; which in a particular embodiment is reduction of the $CO_2$-content of the fuel gas. Simultaneously the combustible component or calorific component of the fuel gas in increased.

It is understood that a method of manufacturing an upgraded gas is considered identical to a method of reducing the $CO_2$ (g) content of a $CO_2$-containing gas and/or reducing or decreasing the partial pressure of $CO_2$ in a $CO_2$-containing gas.

The process that occurs in the bioreactor will allow for the fermentation of the carbon based source whilst consuming the $CO_2$ content of the added fuel gas in order that succinic acid is produced, prior to collecting the upgraded fuel gas and/or the effluent or fermentation broth containing succinic acid.

The upgraded fuel gas may be selected from the group consisting of upgraded biogas, upgraded syngas and upgraded coal gas. The $CO_2$-containing fuel gas may in one embodiment be selected from the group consisting of biogas, syngas and coal gas. A $CO_2$-containing fuel gas is not meant to include substantially pure $CO_2$.

The 'effluent containing succinic acid' as used herein may also be referred to as a liquid phase or fermentation broth. The upgraded fuel gas can be used as a vehicle fuel, or it can be used in a heating power plant, such as a district heating power plant, or it can be used for electricity production. It is a further embodiment to provide a method of manufacturing an upgraded biogas, said method comprising the steps of:

a. providing a bioreactor,
b. providing anaerobic succinic acid-producing organisms,
c. providing a carbon based substrate for said anaerobic succinic acid-producing organisms,
d. adding biogas to the bioreactor,
e. collecting the upgraded biogas thus produced, and
f. optionally collecting the effluent containing succinic acid.

In one embodiment the step of adding biogas to the bioreactor occurs by injecting biogas into the bioreactor, such as injecting the biogas at the bottom of the reactor.

It is understood that a method of manufacturing an upgraded biogas is considered identical to a method of manufacturing a high $CH_4$ content biogas.

The upgraded biogas is preferably upgraded sufficiently in order for it to be utilised in an existing natural gas grid infrastructure and/or directly as a fuel source.

It is understood that a method of manufacturing an upgraded biogas is considered identical to a method of increasing the $CH_4$ content of biogas and/or increasing the partial pressure of $CH_4$ in biogas.

It is understood that a method of manufacturing an upgraded biogas is considered identical to a method of reducing the $CO_2$ (g) content of biogas and/or reducing or decreasing the partial pressure of $CO_2$ in biogas.

By removing the low energy content $CO_2$ from the biogas, the overall energy content of the biogas is increased. Hence it is understood that a method of manufacturing an upgraded biogas is considered identical to a method for increasing the energy content of biogas.

The expression "upgraded biogas" and "high $CH_4$ content biogas" is to be understood as a biogas having a $CH_4$ content exceeding that of conventional non-upgraded biogas. Hence, according to the present disclosure, a "high $CH_4$ content biogas" and "upgraded biogas" typically contains at least 90% $CH_4$, such as at least 91% $CH_4$, for example at least 92% $CH_4$, such as at least 93% $CH_4$, for example at least 94% $CH_4$, such as at least 95% $CH_4$, for example at least 96% $CH_4$, such as at least 97% $CH_4$, for example at least 98% $CH_4$, such as at least 99% $CH_4$, for example at least 99.5% $CH_4$, such as at least 99.6% $CH_4$, for example at least 99.7% $CH_4$, such as at least 99.8% $CH_4$, for example at least 99.9% $CH_4$ as measured by $CH_4$ content of the biogas.

In another embodiment a "high $CH_4$ content biogas" and "upgraded biogas" contains at least between 90-91% $CH_4$, such as 91-92% $CH_4$, for example 92-93% $CH_4$, such as 93-94% $CH_4$, for example 94-95% $CH_4$, such as 95-96% $CH_4$, for example 96-97% $CH_4$, such as 97-98% $CH_4$, for example 98-99% $CH_4$, such as 99-99.5% $CH_4$, for example 99.5-99.9% $CH_4$, such as 99.9-100% $CH_4$, as measured by $CH_4$ content of the biogas.

The biogas produced is preferably of a quality suitable for direct use in the natural gas grid infrastructure without further upgrading/and or purification. It is a further embodiment to provide a method of manufacturing an upgraded syngas or coal gas, said method comprising the steps of:

a. providing a bioreactor,
b. providing anaerobic succinic acid-producing organisms,
c. providing a carbon based substrate for said anaerobic succinic acid-producing organisms,
d. adding syngas or coal gas to the bioreactor,
e. collecting the upgraded syngas or coal gas thus produced, wherein said upgraded syngas or coal gas has a lower content of $CO_2$ than the syngas or coal gas added, and
f. optionally collecting the effluent containing succinic acid.

It is understood that a method of manufacturing an upgraded syngas or coal gas is considered identical to a method of reducing the $CO_2$ content of syngas or coal gas and/or reducing the partial pressure of $CO_2$ in syngas or coal gas.

It is understood that a method of manufacturing an upgraded syngas is considered identical to a method of increasing the $H_2$ and/or CO content of syngas and/or increasing the partial pressure of $H_2$ and/or CO in syngas.

It is understood that a method of manufacturing an upgraded coal gas is considered identical to a method of increasing the $CH_4$, $H_2$ and/or CO content of coal gas and/or increasing the partial pressure of $CH_4$, $H_2$ and/or CO in coal gas. The fuel gas may be produced and upgraded in a single bioreactor, i.e. in situ upgrading, or in separate bioreactors, i.e. ex situ.

It is a further embodiment to provide a method of manufacturing an upgraded fuel gas and simultaneously producing succinic acid, said method comprising the steps of:

a. providing a bioreactor,
b. providing anaerobic succinic acid-producing organisms,
c. providing a carbohydrate substrate for said anaerobic succinic acid-producing organisms,
d. adding a $CO_2$-containing fuel gas to the bioreactor,
e. collecting the upgraded fuel gas thus produced, wherein said upgraded fuel gas has lower $CO_2$ content than the $CO_2$-containing fuel gas added, and
f. collecting the effluent containing succinic acid.

In one embodiment said $CO_2$-containing fuel gas is selected from the group consisting of biogas, coal gas and syngas. In a preferred embodiment of the present disclosure, said $CO_2$-containing gas is biogas.

In one embodiment said $CO_2$-containing gas is not pure $CO_2$. In another said $CO_2$-containing gas comprises at least one other gaseous species other than $CO_2$, such as $H_2$, CO, $CH_4$ and/or $N_2$. Preferably, the $CO_2$-containing gas comprises at least 5%, more preferred at least 10% or more of the at least one other gaseous species.

It is understood that the methods of upgrading a fuel gas and/or producing succinic acid may be optimised in order to achieve the optimum conditions for conversion of a carbon source and $CO_2$ into succinic acid. Initial conditions may be set and lead to the microorganism consuming a carbon source it is fed for the purpose of reproduction, to obtain a well grown cell mass. This cell culture may be obtained in a reactor or container separate from the bioreactor disclosed herein. The cell culture may be transferred into a larger fermenter, the bioreactor, where conditions are set and lead to the microorganism fermenting the carbon source it is fed into the desired product (succinic acid).

In one embodiment the bioreactor is fed with a microorganism culture comprising anaerobic succinic acid-producing microorganisms and a carbon source.

The present inventors have discovered that an increased pressure in the bioreactor can increase the concentration and yield of succinic acid. Thus, in an interesting embodiment fermentation is carried out at a pressure above atmospheric pressure, i.e. above about 1 bar.

In one embodiment the pressure in the bioreactor is at least 1.1 bar, such as at least 1.2 bar, for example at least 1.3 bar, such as least 1.4 bar, for example at least 1.5 bar, such as least 1.6 bar, for example at least 1.7 bar, such as least 1.8 bar, for example at least 1.9 bar, such as least 2.0 bar, for example at least 2.1 bar, such as least 2.2 bar, for example at least 2.3 bar, such as least 2.4 bar, for example at least 2.5 bar, such as least 2.6 bar, for example at least 2.7 bar, such as least 2.8 bar, for example at least 2.9 bar, such as least 3.0 bar, for example at least 3.5 bar, such as least 4 bar, for example at least 5 bar, such as least 6 bar, for example at least 7 bar, such as least 8 bar or more.

In one embodiment the pressure in the bioreactor is between 1.1 and 10 bar, such as between 1.1 and 5 bar, for example between 1.1 and 4 bar, such as between 1.1 and 3 bar, for example between 1.1 and 2 bar.

In one embodiment the pressure in the bioreactor is between 1.2 and 10 bar, such as between 1.2 and 5 bar, for example between 1.2 and 4 bar, such as between 1.2 and 3 bar, for example between 1.2 and 2 bar.

In one embodiment the pressure in the bioreactor is between 1.3 and 10 bar, such as between 1.3 and 5 bar, for example between 1.3 and 4 bar, such as between 1.3 and 3 bar, for example between 1.3 and 2 bar.

In one embodiment the pressure in the bioreactor is between 1.4 and 10 bar, such as between 1.4 and 5 bar, for example between 1.4 and 4 bar, such as between 1.4 and 3 bar, for example between 1.4 and 2 bar.

In one embodiment the pressure in the bioreactor is between 1.5 and 10 bar, such as between 1.5 and 5 bar, for example between 1.5 and 4 bar, such as between 1.5 and 3 bar, for example between 1.5 and 2 bar.

*Succinic acid producing organism*

The succinic acid production process will be catalyzed or mediated by anaerobic succinic acid-producing organisms, preferably microorganisms, which will utilize $CO_2$ contained in a fuel gas, such as the $CO_2$ from biogas. In this way the fuel gas is upgraded by the succinic acid fermentation process, in the case of biogas thus obtaining high purity biomethane.

Anaerobic succinic acid-producing organisms include any organism that is able to fixate $CO_2$ under anaerobic conditions thereby promoting the production of succinic acid.

Anaerobic succinic acid-producing organisms include any organism that is capable of fermenting a carbon source to succinic acid.

Anaerobic succinic acid-producing organisms are in one embodiment anaerobic succinic acid-producing microorganisms.

Anaerobic succinic acid-producing organisms or microorganisms are in one embodiment anaerobic succinic acid-producing bacteria.

Anaerobic succinic acid-producing bacteria include any bacteria that are able to fixate $CO_2$ under anaerobic conditions thereby promoting the production of succinic acid.

Anaerobic succinic acid-producing bacteria include any bacteria that are able to produce succinic acid under physiological conditions in the presence of a carbon based substrate and $CO_2$; such as glucose and $CO_2$.

In one embodiment said anaerobic succinic acid-producing bacteria is selected from the group consisting of *Actinobacillus succinogenes (A. succinogenes), Anaerobiospirillum succiniciproducens (A. succiniciproducens), Mannheimia succiniciproducens (M. succiniciproducens), Corynebacterium glutamicum (C. glutamicum)* and recombinant *Escherichia coli (E. coli)*.

In one particular embodiment said anaerobic succinic acid-producing bacteria is *Actinobacillus succinogenes.*

*Actinobacillus succinogenes* substrates and the metabolic pathway of *Actinobacillus succinogenes* is disclosed in McKinlay et al. 2010.

Osmophilic organisms are microorganisms adapted to environments with high osmotic pressures, such as high sugar concentrations. *A.succinogenes* is a moderate osmophile and has a high tolerance to initial sugar concentrations (≤158 g/L glucose) and also a high succinic acid concentrations (>70 g/L).

In one embodiment said anaerobic succinic acid-producing microorganism is an engineered (or recombinant) microorganism capable of fermenting a carbon source to succinic acid.

In the embodiment wherein the anaerobic succinic acid-producing bacteria is recombinant *E. coli,* said recombinant *E. coli* is modified in order to be capable of fermenting a carbon source to succinic acid. E. coli may e.g. be modified as previously described in Wu et al. 2007 by genetic manipulation of central metabolic pathways to enhance succinic acid production. For example, one strategy is to block the competition pathways to succinic acid, such as by inactivation of pyruvate:formate lyase (PFL) and lactate dehydrogenase (LDH), the protein EIICB in the phophotransferase system (PTS), alcohol dehydrogenase (ADH), phosphate acetyltransferase-acetate kinase, etc. Another strategy is to overexpress endogenous or heterologous $CO_2$ fixing enzymes, such as pyruvate carboxylase, phosphoenolpyruvate (PEP) carboxylase (PPC), PEP carboxykinase (PCK) and malic enzyme (ME).

*Carbon based substrate*

A substrate for an anaerobic succinic acid-producing organism or microorganism, such as bacteria, is a substrate required and sufficient for the organism to fixate $CO_2$ under anaerobic conditions to promote the production of succinic acid.

In one embodiment said substrate is a carbohydrate substrate.

In one embodiment said substrate is a high carbohydrate content substrate.

In general, any and all types of hydrolysed biomass may be used as substrate as long as the carbohydrate content in the hydrolysate is high.

Some industrial waste products have high carbohydrate content and may thus be used as a substrate according to the present disclosure. Thus in one embodiment the substrate is an industrial waste product such as an agricultural, forest and/or a food industry waste. Examples of suitable waste products include glycerol waste from the biodiesel industry, whey from cheese production and pretreated agriculture and forest waste such as straw, woodchips, animal dung including e.g. cow or horse dung.

Energy crops are also contemplated as possible substrates. An energy crop is a plant grown as a low-cost and low-maintenance harvest conventionally used to make biofuels, such as bioethanol, or combusted for its energy content to generate electricity or heat. Energy crops are generally categorized as woody or herbaceous plants; many of the latter are grasses (Graminaceae). Commercial energy crops are typically densely planted, high-yielding crop species where the energy crops will be burnt to generate power. Woody crops such as willow or poplar are widely utilised, as well as temperate grasses such as Miscanthus and Pennisetum purpureum. Whole-crops such as maize, Sudan grass, millet, white sweet clover and many others are also contemplated as possible carbohydrate substrates. The energy crops may be pretreated (hydrolysed) to obtain a higher amount of fermentable sugars.

In one embodiment said carbon based or carbohydrate substrate comprises or consists of one or more of glucose, xylose, arabinose, galactose, maltose, fructose, sucrose, cellobiose, lactose, mannitol, arabitol, sorbitol, mannose, ribose, glycerol, pectin, beta-glucoside, gluconate, idonate, ascorbate, glucarate, galactarate and/or 5-keto-glucanate.

In one embodiment said carbohydrate substrate comprises one or more fermentable sugars. In one embodiment of the present disclosure, said substrate is a fermentable carbohydrate substrate.

In one embodiment said carbohydrate substrate comprises one or more of glucose, xylose, arabinose, galactose, maltose, fructose, sucrose, cellobiose, lactose, mannitol, arabitol, sorbitol, mannose and/or ribose.

In one particular embodiment said carbohydrate substrate comprises glucose.

In one embodiment said carbohydrate substrate comprises glycerol.

In another particular embodiment said carbohydrate substrate comprises a reduced sugar and/or a sugar alcohol (i.e. derived from a sugar by reduction). In one embodiment of the present disclosure, said substrate comprises or consists of one or more of mannitol, arabitol and/or sorbitol.

In one embodiment carbohydrate substrate is a mixture of carbohydrates comprising at least two different carbohydrates, such as at least three different carbohydrates, for example at least four different carbohydrates, such as at least five different carbohydrates or more.

*Bioreactor*

Preferably, the reactions will take place in a bioreactor suitable therefore, such as a bioreactor capable of containing gas and providing a substantially anaerobic milieu.

The methods for upgrading biogas is particularly efficient if a bioreactor is used which comprises a gas injection system capable of injecting $CO_2$-containing gas into the fluid phase of the bioreactor content, without causing bubbles. A suitable bioreactor comprises:

  a. a reactor vessel
  b. a gas injection system
  c. loading means suitable for adding to the vessel a succinic acid producing organism and one or more carbohydrate substrates, and
  d. discharge means for products and/or waste.

Said reactor vessel can comprise a liquid phase, wherein said liquid phase may be a fermentation broth.

The reactor vessel of the bioreactor preferably is a closed vessel capable of containing gas and providing a substantially anaerobic milieu. In one embodiment of the method a continuous stirred-tank reactor (CSTR) is made of use.

The process of upgrading fuel gas such as biogas and/or producing succinic acid may be made as a batch process, whereby the components are loaded into the vessel of the bioreactor, the reaction proceeds, and the vessel emptied.

The process of upgrading gas such as biogas and/or producing succinic acid may be made as a continuous process, whereby the components are continuously loaded into and extracted from the vessel of the bioreactor.

The gas injection system is used to introduce the $CO_2$ containing fuel gas into the bioreactor.

In one embodiment of the method the $CO_2$ containing fuel gas is added continuously to the bioreactor. Preferably, said gas injection system is located at the bottom of the reactor or vessel, in order that the gas may be injected at the bottom of the reactor. This will allow addition of the gas directly into the liquid phase or fermentation broth comprised in the reactor vessel.

Preferably the size of the gas or biogas bubbles so injected is maintained minimal to keep their surface area per volume as high as possible, in order that the $CO_2$ of the gas can diffuse more freely into the liquid phase comprising succinic acid producing organisms and a carbon based source.

According to Henry's law ($[CO2] = P_{CO2}/H_0$) there is a balance between $CO_2$ dissolved in liquid and $CO_2$ in gas phase. As the biogas bubbles go through the reactor and the $CO_2$ is consumed the partial pressure of $CO_2$ decreases in the liquid phase which forces $CO_2$ of gas phase to be dissolved into the liquid. By the time the bubbles reach the headspace above the liquid substantially all $CO_2$ has been removed by the succinic acid producing organism, leaving purified methane. As methane also is far less soluble in water than $CO_2$ little methane will diffuse from the bubble into the liquid.

Bubbleless gas-transfer via hollow fibre membrane (HFM) is a potential method to achieve the purpose of complete utilization of $CO_2$. Gas inside the HFM can be delivered into the liquid phase through membranes by diffusion. Thus, the gas is directly dissolved in the liquid, no bubble is formed and the $CO_2$ is taken up by the microorganisms more efficiently. Thus, in a particular embodiment of the method said gas injection system comprises hollow fibres to introduce the $CO_2$-containing fuel gas into the bioreactor.

The loading means suitable for adding to the vessel a succinic acid producing organism and one or more carbon based substrates may be capable of providing said components, feeding said bioreactor with said components, adding and/or injecting said components into the bioreactor. The bioreactor comprises a $CO_2$ containing fuel gas as defined herein, such as biogas, syngas, or coal gas; preferably biogas.

In one embodiment of the method the bioreactor comprises succinic acid producing bacteria and one or more carbohydrate sources, which are described herein elsewhere.

The optimal conditions for the fermentation reaction may be determined by the skilled person, including variable factors such as flow rate, temperature, pH etc.

Preferably the biogas flow rate into the reactor is balanced with the rate of $CO_2$ and carbohydrate consumption.

In one embodiment of the method, the carbon based substrate and the succinic acid producing organisms are mixed and added to the bioreactor together as a mixture; in another embodiment the carbon based substrate is fed to the reactor separately from the succinic acid producing bacteria.

*Recovery of succinic acid from effluent*

The method may comprise collecting the effluent of the bioreactor containing succinic acid. Said effluent refers to the liquid phase contained in the reactor, and may also be denoted a fermentation broth.

Further to the fermentation the succinic acid is separated and recovered from the fermentation broth where it is present as a dissolved salt (succinate), along with the microorganism biomass, non-converted sugars, small quantity of by-products, and other remains of the medium.

In one embodiment the method comprises an additional step of recovering and/or processing the effluent containing succinic acid, whereby said succinic acid may be isolated, concentrated, purified and/or recovered.

In one embodiment the method further comprises the step of recovering and/or purifying succinic acid from the effluent containing succinic acid. Thus a recovery and purification step may be employed to obtain pure succinic acid (crystals) meeting downstream specification requirements for derivatives production. One method for doing so is presented herein, but other methods may be envisaged. In one embodiment of method the first downstream processing step is cell separation by centrifugal separation or microfiltration, usually followed by ultrafiltration to separate cell debris, proteins and other polymers from the fermentation supernatant.

For the isolation and concentration of succinic acid many unit operations have been suggested: precipitation with ammonia or calcium hydroxide, membrane processes such as electrodialysis, liquid-liquid extraction such as predispersed solvent extraction with colloidal liquid aphrons, reactive extraction, distillation, counter current extraction, esterification, chromatography etc. Different sorption/ion exchange methods and crystallization are the methods of choice for the final purification of the isolated acid. Advantages and disadvantages of all these operations are discussed in Kurzrock & Weuster-Botz.

**References**

Zeikus, J.G et al. 1999. Biotechnology of succinic acid production markets for derived industrial products. Appl. Microbiol. Biotechnol. 51, 545-552.

Kurzrock & Weuster-Botz. 2010. Recovery of succinic acid from fermentation broth. Biotechnol. Lett. 32, 331-339.

McKinlay et al. "A genomic perspective on the potential of Actinobacillus succinogenes for industrial succinate production". BMC Genomics 2010, 11:680.

Song & Lee, "Production of succinic acid by bacterial fermentation," Enzyme and Microbial Technology, vol. 39, no. 3, pp. 352-361, Jul. 2006.

Lin, et al. "Substrate and product inhibition kinetics in succinic acid production by Actinobacillus succinogenes," Biochemical Engineering Journal, vol. 41, no. 2, pp. 128-135, Sep. 2008.

Guettler et al, "Actinobacillus succinogenes sp. nov., a novel succinic-acid-producing strain from the bovine rumen," Int J Syst Bacteriol, vol. 49, no. 1, pp. 207-216, Sep. 1999.

Airliquide, "Gas Encyclopaedia," 2009. [Online]. Available: http://encyclopedia.airliquide.com/encyclopedia.asp.

Petersson & Wellinger "Biogas upgrading technologies - developments and innovations," 2009.

Ryckebosch et al. "Techniques for transformation of biogas to biomethane", Bimass and Bioenergy, vol. 35, no. 5, pp. 1633-1645, May 2011.

Wu et al. "Improved Succinic Acid Production in the Anaerobic Culture of an Escherichia coli pflB ldhA Double Mutant as a Result of Enhanced Anaplerotic Activities in the Preceding Aerobic Culture", Appl Environ Microbiol, pp. 7837-7843, Dec 2007.

**Examples**

*Experimental setup:*

3-L bioreactors (Sartorius BIOSTAT Aplus, Germany) with an initial working volume of 1.5 L were used. The bioreactors were equipped with EASYFERM PLUS K8 200 pH probes (Hamilton Bonaduz AG, Switzerland), temperature and $CO_2$ (Mettler Toledo, Switzerland). Flowrate of gas mixtures was controlled through a peristaltic pump (Watson-Marlow, United Kingdom). For monitoring and controlling pressure inside bioreactors a pressure gauge (Cewai, Italy) was mounted with a relief valve (Hy-Lok, USA) set to the experimental pressure. Different size gas bags (6.7, 7.5 and 12.5 L) were used to supply gas to the system. The system consists of a gas recirculation which allows observing changes in biogas composition during fermentation. Experiments have been carried out using both $MgCO_3$ (supplies excess $CO_2$ during fermentation) and biogas (60% $CH_4$ / 40% $CO_2$).

Microorganism, medium and succinic acid fermentation

The strain *A. succinogenes* 130Z was obtained from DSMZ. The culture stock was stored in glycerol at -80°C until used. Seed culture medium was composed of (gr. per litre): glucose (10.0), yeast extract (5.0), $NaHCO_3$ (10.0), $NaH_2PO_4\cdot 2H_2O$ (9.6), $K_2HPO_4\cdot 3H_2O$ (20.3). Medium was sterilized at 121 °C for 20 min. Seed culture was cultivated at 37°C and 150 rpm in 50 mL sealed anaerobic bottles containing 30 ml medium and inoculated with 1 ml of -80 glycerol stock culture.

Biogas upgrading and succinic acid production batch experiments were conducted in duplicates. Fermentation experiments were conducted using biogas (60% CH4, 40% CO2) or 100% CO2 as source of CO2 for succinic acid production. Fermentations were carried out at either 1.01325 bar (atmospheric pressure) or 1.6 bar pressure.

Experimental medium for biogas upgrading and fermentation was composed of (per litre): yeast extract (10.0), $K_2HPO_4$ (3.0), $MgCl_2$ (0.2), $CaCl_2$ (0.2), NaCl (1.0). Medium was sterilized at 121 °C for 20 min.

All batch fermentations in 3-l bioreactors were carried out at 37°C, pH 6.75, 200 rpm for 24 hours and inoculated with 5% (v/v) of exponentially growing inoculum. Prior to start of fermentation, pH was adjusted to 6.8 using 50% phosphoric acid and 0.05ml of sterile Antifoam 204 (Sigma Aldrich) was added. 8 M NaOH was added automatically to maintain pH at 6.8 during fermentation. $N_2$ gas was used in all cases to create anaerobic conditions.

*Sample analysis:*

Glucose, succinate, lactate, formate, acetate and ethanol were measured and quantified by using High Performance Liquid Chromatography (HPLC). The HPLC (Agilent) had a refractive index detector, a Bio-Rad Aminex HPX-87H column (300 mm $\times$ 7.8 mm) with 0.04 M $H_2SO_4$ as eluent and flow rate was set to 0.6 mL/min with column oven temperature set to 63.5°C.

Methane and carbon dioxide were analyzed with GC-TCD fitted with parallel column of 1.1 m $\times$ 3/16 in. Molsieve 137 and 0.7 m $\times$ 1/4 in. chromosorb 108.

Soluble $CO_2$ was monitored by using Mettler Toledo InPro 5000 CO2 sensor / $CO_2$ 5100 e transmitter. Bacterial growth was measured at 660nm using Jenway Buch and Holm A/S 64050UV/vis spectrophotometer.

The results of the experiments are shown in figures 2 to 6.

*Conclusion:*

The present inventors have shown that biogas can be upgraded to contain more than 99% (99.6%) $CH_4$ using the present method comprising simultaneous production of succinic acid production.

**Claims**

1. A method of manufacturing an upgraded fuel gas, said method comprising the steps of:

   a. providing a bioreactor,
   b. providing anaerobic succinic acid-producing microorganisms,
   c. providing a carbon based substrate for said anaerobic succinic acid-producing microorganisms,
   d. adding a $CO_2$-containing fuel gas to the bioreactor,
   e. collecting the upgraded fuel gas thus produced, wherein said upgraded fuel gas has a lower $CO_2$ content than the $CO_2$-containing fuel gas added, and

f. optionally collecting the effluent containing succinic acid.

2. A method of manufacturing an upgraded fuel gas and simultaneously producing succinic acid, said method comprising the steps of:

    a. providing a bioreactor,
    b. providing anaerobic succinic acid-producing microorganisms,
    c. providing a carbon based substrate for said anaerobic succinic acid-producing microorganisms,
    d. adding a $CO_2$-containing fuel gas to the bioreactor,
    e. collecting the upgraded fuel gas thus produced, wherein said upgraded fuel gas has a lower $CO_2$ content than the $CO_2$-containing fuel gas added, and
    f. collecting the effluent containing succinic acid.

3. The method of any of the preceding claims, wherein said $CO_2$-containing fuel gas is selected from the group consisting of biogas, syngas and coal gas.

4. The method of any of the preceding claims, wherein said $CO_2$-containing fuel gas is biogas.

5. The method of claim 4, wherein the upgraded biogas thus produced has a $CH_4$ content of at least 90% $CH_4$.

6. The method of any of the preceding claims, wherein said anaerobic succinic acid-producing microorganism is an anaerobic succinic acid-producing bacteria.

7. The method of claim 6, wherein said anaerobic succinic acid-producing bacteria is selected from the group consisting of *Actinobacillus succinogenes (A. succinogenes), Anaerobiospirillum succiniciproducens (A. succiniciproducens), Mannheimia succiniciproducens (M. succiniciproducens), Corynebacterium glutamicum (C. glutamicum)* and recombinant *Escherichia coli (E. coli).*

8. The method of any of claims 6 to 7, wherein said anaerobic succinic acid-producing bacteria is *Actinobacillus succinogenes.*

9. The method of any of the preceding claims, wherein said carbon based substrate is a carbohydrate substrate.

10. The method of claim 9, wherein said carbohydrate substrate comprises one or more of glucose, xylose, arabinose, galactose, maltose, fructose, sucrose, cellobiose, lactose, mannitol, arabitol, sorbitol, mannose, ribose, glycerol, pectin, beta-glucoside, gluconate, idonate, ascorbate, glucarate, galactarate and/or 5-keto-glucanate.

11. The method of claim 10, wherein the carbohydrate substrate comprises glycerol.

12. The method of any of the preceding claims, wherein said method further comprises the step of recovering and/or purifying succinic acid from the effluent containing succinic acid.

13. The method of any of the preceding claims, wherein said bioreactor comprises a gas injection system comprising hollow fibres.

14. The method of any of the preceding claims, wherein said bioreactor is a continuous stirred-tank reactor (CSTR).

**Patentansprüche**

1. Verfahren zur Herstellung eines veredelten Brenngases, wobei das Verfahren die folgenden Schritte umfasst:

    a. Bereitstellen eines Bioreaktors,
    b. Bereitstellen von anaeroben bernsteinsäureproduzierenden Mikroorganismen,
    c. Bereitstellen eines kohlenstoffbasierenden Substrats für die anaeroben bernsteinsäureproduzierenden Mikroorganismen,
    d. Hinzufügen eines $CO_2$-haltigen Brenngases zu dem Bioreaktor,
    e. Sammeln des dadurch produzierten veredelten Brenngases, wobei das veredelte Brenngas einen geringeren

CO$_2$-Gehalt als das hinzugefügte CO$_2$-haltige Brenngas aufweist, und

f. optional Sammeln des Abflusses, der Bernsteinsäure enthält.

**2.** Verfahren zur Herstellung eines veredelten Brenngases und simultanen Produktion von Bernsteinsäure, wobei das Verfahren die folgenden Schritte umfasst:

a. Bereitstellen eines Bioreaktors,
b. Bereitstellen von anaeroben bernsteinsäureproduzierenden Mikroorganismen,
c. Bereitstellen eines kohlenstoffbasierenden Substrats für die anaeroben bernsteinsäureproduzierenden Mikroorganismen,
d. Hinzufügen eines CO$_2$-haltigen Brenngases zu dem Bioreaktor,
e. Sammeln des dadurch produzierten veredelten Brenngases, wobei das veredelte Brenngas einen geringeren CO$_2$-Gehalt als das hinzugefügte CO$_2$-haltige Brenngas aufweist, und
f. Sammeln des Abflusses, der Bernsteinsäure enthält.

**3.** Verfahren nach einem der vorhergehenden Ansprüche, wobei das CO$_2$-haltige Brenngas aus der Gruppe ausgewählt ist, die aus Biogas, Synthesegas und Kohlengas besteht.

**4.** Verfahren nach einem der vorhergehenden Ansprüche, wobei das CO$_2$-haltige Brenngas Biogas ist.

**5.** Verfahren nach Anspruch 4, wobei das dadurch produzierte veredelte Biogas einen CH$_4$-Gehalt von mindestens 90 % CH$_4$ aufweist.

**6.** Verfahren nach einem der vorhergehenden Ansprüche, wobei der anaerobe bernsteinsäureproduzierende Mikroorganismus eine anaerobe bernsteinsäureproduzierende Bakterie ist.

**7.** Verfahren nach Anspruch 6, wobei die anaerobe bernsteinsäureproduzierende Bakterie aus der Gruppe ausgewählt ist, die aus *Actinobacillus succinogenes (A. succinogenes)*, *Anaerobiospirillum succiniciproducens (A. succiniciproducens)*, *Mannheimia succiniciproducens (M. succiniciproducens)*, *Corynebacterium glutamicum (C. glutamicum)* und rekombinantem *Escherichia coli (E. coli)* besteht.

**8.** Verfahren nach einem der Ansprüche 6 bis 7, wobei die anaerobe bernsteinsäureproduzierende Bakterie *Actinobacillus succinogenes* ist.

**9.** Verfahren nach einem der vorhergehenden Ansprüche, wobei das kohlenstoffbasierte Substrat ein Kohlenhydratsubstrat ist.

**10.** Verfahren nach Anspruch 9, wobei das Kohlenhydratsubstrat eines oder mehrere von Glucose, Xylose, Arabinose, Galactose, Maltose, Fructose, Sucrose, Cellobiose, Lactose, Mannitol, Arabitol, Sorbitol, Mannose, Ribose, Glycerol, Pectin, beta-Glucosid, Gluconat, Idonat, Ascorbat, Glucarat, Galactarat und/oder 5-keto-Glucanat umfasst.

**11.** Verfahren nach Anspruch 10, wobei das Kohlenhydratsubstrat Glycerol umfasst.

**12.** Verfahren nach einem der vorhergehenden Ansprüche, wobei das Verfahren ferner den Schritt des Gewinnens und/oder Reinigens von Bernsteinsäure aus dem Abfluss, der Bernsteinsäure enthält, umfasst.

**13.** Verfahren nach einem der vorhergehenden Ansprüche, wobei der Bioreaktor ein Gaseinspritzsystem umfasst, das Hohlfasern umfasst.

**14.** Verfahren nach einem der vorhergehenden Ansprüche, wobei der Bioreaktor ein kontinuierlich gerührter Tankreaktor (CSTR) ist.

**Revendications**

**1.** Procédé de fabrication d'un gaz combustible valorisé, ledit procédé comprenant les étapes suivantes :

a. la fourniture d'un bioréacteur,

b. la fourniture de micro-organismes anaérobies produisant de l'acide succinique,

c. la fourniture d'un substrat à base de carbone pour lesdits micro-organismes anaérobies produisant de l'acide succinique,

d. l'ajout d'un gaz combustible contenant du $CO_2$ au bioréacteur,

e. la collecte du gaz combustible valorisé ainsi produit, dans lequel ledit gaz combustible valorisé présente une teneur en $CO_2$ plus basse que le gaz combustible contenant du $CO_2$ ajouté, et

f. facultativement la collecte de l'effluent contenant de l'acide succinique.

2. Procédé de fabrication d'un gaz combustible valorisé et de production simultanée d'acide succinique, ledit procédé comprenant les étapes suivantes :

a. la fourniture d'un bioréacteur,

b. la fourniture de micro-organismes anaérobies produisant de l'acide succinique,

c. la fourniture d'un substrat à base de carbone pour lesdits micro-organismes anaérobies produisant de l'acide succinique,

d. l'ajout d'un gaz combustible contenant du $CO_2$ au bioréacteur,

e. la collecte du gaz combustible valorisé ainsi produit, dans lequel ledit gaz combustible valorisé présente une teneur en $CO_2$ plus basse que le gaz combustible contenant du $CO_2$ ajouté, et

f. la collecte de l'effluent contenant de l'acide succinique.

3. Procédé selon l'une quelconque des revendications précédentes, dans lequel ledit gaz combustible contenant du $CO_2$ est sélectionné dans le groupe constitué d'un biogaz, d'un gaz de synthèse et d'un gaz de houille.

4. Procédé selon l'une quelconque des revendications précédentes, dans lequel ledit gaz combustible contenant du $CO_2$ est un biogaz.

5. Procédé selon la revendication 4, dans lequel le biogaz valorisé ainsi produit présente une teneur en $CH_4$ d'au moins 90 % de $CH_4$.

6. Procédé selon l'une quelconque des revendications précédentes, dans lequel ledit micro-organisme anaérobie produisant de l'acide succinique est une bactérie anaérobie produisant de l'acide succinique.

7. Procédé selon la revendication 6, dans lequel ladite bactérie anaérobie produisant de l'acide succinique est sélectionnée dans le groupe constitué de *Actinobacillus succinogenes* (*A. succinogenes*), *Anaerobiospirillum succiniciproducens* (*A. succiniciproducens*), *Mannheimia succiniciproducens* (*M. succiniciproducens*), *Corynebacterium glutamicum* (*C. glutamicum*) et d'une *Escherichia coli* recombinante (*E. coli*).

8. Procédé selon l'une quelconque des revendications 6 à 7, dans lequel ladite bactérie anaérobie produisant de l'acide succinique est *Actinobacillus succinogenes*.

9. Procédé selon l'une quelconque des revendications précédentes, dans lequel ledit substrat à base de carbone est un substrat glucidique.

10. Procédé selon la revendication 9, dans lequel ledit substrat glucidique comprend un ou plusieurs parmi le glucose, le xylose, l'arabinose, le galactose, le maltose, le fructose, le saccharose, le cellobiose, le lactose, le mannitol, l'arabitol, le sorbitol, le mannose, le ribose, le glycérol, la pectine, un bêta-glucoside, un gluconate, un idonate, un ascorbate, un glucarate, un galactarate et/ou un 5-céto-glucanate.

11. Procédé selon la revendication 10, dans lequel le substrat glucidique comprend du glycérol.

12. Procédé selon l'une quelconque des revendications précédentes, dans lequel ledit procédé comprend en outre l'étape de récupération et/ou de purification de l'acide succinique provenant de l'effluent contenant de l'acide succinique.

13. Procédé selon l'une quelconque des revendications précédentes, dans lequel ledit bioréacteur comprend un système d'injection de gaz comprenant des fibres creuses.

14. Procédé selon l'une quelconque des revendications précédentes, dans lequel ledit bioréacteur est un réacteur à cuve sous agitation continue (CSTR).

**Figure 1**

ANY REFERENCE TO FIGURES 1A, 1B, 1C AND 1D SHALL BE CONSIDERED AS NON-EXISTENT

**Figure 2**

**Figure 3**

**Figure 4**

**Figure 5**

**Figure 6**

**Figure 7**

**Figure 8**

**Figure 8 - continued**

**Figure 9**

**Figure 9 - continued**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013060331 A

**Non-patent literature cited in the description**

- **NGHIEM et al.** *Applied Biochemistry and Biotechnology,* 2010, vol. 162, 1915-1928
- **LUO et al.** *Biotechnology and Bioengineering,* 2012, vol. 109, 2729-2736
- **LU et al.** *30th Symposium on Biotechnology for Fuels and Chemicals,* 2008
- **GUNNARSSON et al.** *Afternoon Seminar on Biorefinery in the Oresund region,* June 2013
- **ZEIKUS, J.G et al.** Biotechnology of succinic acid production markets for derived industrial products. *Appl. Microbiol. Biotechnol.,* 1999, vol. 51, 545-552
- **KURZROCK ; WEUSTER-BOTZ.** Recovery of succinic acid from fermentation broth. *Biotechnol. Lett.,* 2010, vol. 32, 331-339
- **MCKINLAY et al.** A genomic perspective on the potential of Actinobacillus succinogenes for industrial succinate production. *BMC Genomics,* 2010, vol. 11, 680
- **SONG ; LEE.** Production of succinic acid by bacterial fermentation. *Enzyme and Microbial Technology,* July 2006, vol. 39 (3), 352-361
- **LIN et al.** Substrate and product inhibition kinetics in succinic acid production by Actinobacillus succinogenes. *Biochemical Engineering Journal,* September 2008, vol. 41 (2), 128-135
- **GUETTLER et al.** Actinobacillus succinogenes sp. nov., a novel succinic-acid-producing strain from the bovine rumen. *Int J Syst Bacteriol,* September 1999, vol. 49 (1), 207-216
- Airliquide. Gas Encyclopaedia. 2009
- **PETERSSON ; WELLINGER.** *Biogas upgrading technologies - developments and innovations,* 2009
- **RYCKEBOSCH et al.** Techniques for transformation of biogas to biomethane. *Bimass and Bioenergy,* May 2011, vol. 35 (5), 1633-1645
- **WU et al.** Improved Succinic Acid Production in the Anaerobic Culture of an Escherichia coli pflB ldhA Double Mutant as a Result of Enhanced Anaplerotic Activities in the Preceding Aerobic Culture. *Appl Environ Microbiol,* December 2007, 7837-7843